Europäisches Patentamt

European Patent Office

Office européen des brevets

(11) Publication number: **0 357 327**
**A2**

## (12) EUROPEAN PATENT APPLICATION

(21) Application number: **89308529.0**

(22) Date of filing: **23.08.89**

(51) Int. Cl.⁵: **A 61 K 9/22**

(30) Priority: **25.08.88 GB 8820242**

(43) Date of publication of application:
**07.03.90 Bulletin 90/10**

(84) Designated Contracting States: **DE FR GB IT NL**

(71) Applicant: **NATIONAL RESEARCH DEVELOPMENT CORPORATION**
**101 Newington Causeway**
**London SE1 6BU (GB)**

(72) Inventor: **Kent, Brian Ernest**
**126 Brighton Road**
**Tadworth Surrey KT20 6AQ (GB)**

**Prosser, Havard John**
**94 Green Drift**
**Royston Hertfordshire SG8 5BT (GB)**

**Wilson, Alan Donald**
**49 Shepherds Way**
**Liphook Hampshire GU30 7HH (GB)**

(74) Representative: **Neville, Peter Warwick**
**Patent Department National Research Development Corporation 101 Newington Causeway**
**London SE1 6BU (GB)**

(54) Sustained release device.

(57) An acid-base reaction cement of magnesium oxide base plus magnesium sulphate as acid includes amprolium and optionally a weighting agent, and is formed into a bolus or other sustained-release device for oral administration to cows. The rate of release of amprolium is convenient for husbandry.

Fig. 2

**Description**

## SUSTAINED RELEASE DEVICE

This invention relates to sustained release devices; more particularly, this invention relates to such devices which provide sustained release of active substances, especially substances having a medicinal effect. The invention is of particular, but not exclusive, relevance to animal husbandry.

During the last half-century it has become increasingly clear that, in order to maintain a complete, balanced metabolism, healthy livestock requires trace but sustained amounts of a number of chemical elements. Furthermore, ever more intensive systems of animal husbandry dictate that these requirements have to be rigorously adhered to.

This invention seeks to provide a device which can give sustained release of one or more active substances, especially substances having a medicinal effect in a form preferably, but not necessarily, for oral administration or parenteral implant, not requiring frequent dosing.

The device may be a shaped cementitious mass or of crushed cementitious material formed into a tablet.

UK Patent GB 2123693B discloses and claims a sustained release device which comprises a source of at least one trace chemical element, optionally with at least one active substance other than a trace chemical element, incorporated in a cement which comprises a coherent mass formed by reaction between a Bronsted acid and a base and releasable therefrom on contact with an aqueous medium. At least one trace chemical element may be an intrinisic component of the cement and/or at least one trace chemical element may be dispersed in the cement.

Such devices have also been found suitable for uses other than in oral administration or parenteral implant in livestock and where sustained release is desired, for example in the provision of one or more active substances in agriculture or horticulture.

According to the present invention, a sustained release device comprises a shaped cementitious mass or a tablet formed of crushed cementitious material, the cement being an acid-base reaction cement and comprising a pharmaceutically active organic substance. An acid-base reaction cement is one in which one cement-forming component is a Bronsted acid such as phosphoric acid (50-90%), poly(acrylic acid) (50.0%), malic acid (50.0%), tricarballylic acid (50.0%), pyruvic acids (50.0%), tartaric acid (50.0%), mellitic acid (50.0%), gallic acid (50.0%), tannic acid (50.0%) or a Lewis acid such as magnesium chloride (35%), zinc chloride (80%), copper (II) chloride (40.0%), cobalt (II) chloride (sat), magnesium sulphate (20.0%), zinc sulphate (30.0%), copper (II) sulphate (12.0%), cobalt (II) sulphate (20.0%), magnesium selenate (sat), zinc selenate (50.0%), copper (II) selenate (25.0%), cobalt (II) selenate (50.0%), or salicylic ester; and another cement-forming component is a Bronsted base such as copper (II) oxide, zinc (II) oxide, magnesium oxide, cobalt (II) hydroxide, cobalt (II) carbonate, calcium aluminosilicate glasses, gelatinizing minerals, or other aluminosilicate glass or calcium hydroxide.

Thus, more generally, in the preferred case the cement is suitably formed from an acid cement-forming component which comprises a mineral acid, an acid salt, a polyfunctional organic carboxylic acid, a polyfunctional organic phosphoric acid, a polyfunctional phenol, a homo- or co-polymer of an unsaturated carboxylic acid, a homo- or co-polymer of an unsaturated sulphonic acid, or a hydrolysable precursor thereof. The term "hydrolysable precursor" as used herein includes any species, such as an anhydride or an acid chloride, which is transformed on hydrolysis to the required acid cement-forming component. Suitable examples of mineral acids include phosphoric acids such as orthophosphoric acid, pyrophosphoric acid and meta-phosphoric acids, sulphuric acid, nitric acid, and hydrohalic acids, such as hydrochloric acid, with phosphoric acids being preferred. Examples of acid salts include the hydrogen and dihydrogen phosphates; bisulphates and bifluorides, especially the alkali metal hydrogen and dihydrogen phosphates. Examples of Lewis acids of such may be incorporated with the Bronsted acids, include those metal halides which are Lewis acids, such as aluminium trichloride, aluminium tribromide, magnesium chloride, magnesium iodide, ferric chloride, zinc chloride, zinc iodide and copper chloride. Others are oxyacid salts, such as sulphates, selenates and selenites. Examples of polyfunctional organic carboxylic acids, polyfunctional organic phosphoric acids and polyfunctional phenols include the following polybasic acids; malonic, mesoxalic, succinic, glutaric, adipic, pimelic, suberic, azelaic, sebacic, malic, citric, tartaric, tartronic, tricarballylic, maleic, fumaric, citraconic, mesaconic, itaconic, glutaconic, muconic, aconitic, ortho-, iso- and tere-phthalic, gallic, tannic and mellitic acids, phytic acid, catechol, resorcinol, quinol, pyrogallol, hydroxyquinol and phloroglucinol. Other polyfunctional organic carboxylic acids and phenols which are not polybasic but are suitable as acid cement-forming components include hydroxycarboxylic acids and ketoacids. Examples are lactic, pyruvic, 2-hydroxyisobutyric, 2-hydroxycyclohexane carboxylic, 2-hydroxy-2-phenyl propionic, diphenylhydroxyacetic, 2-hydroxybenzoic, 3-hydroxybenzoic and 4-hydroxybenzoic acids, eugenol and silicylaldehyde. Examples of homo- or co-polymers of an unsaturated carboxylic acid include those prepared by the homopolymerisation or copolymerisation of aconitic acid, acrylic acid, citraconic acid, fumaric acid, glutaconic acid, itaconic acid, maleic acid, mesaconic acid, methacrylic acid, muconic acid and tiglic acid, and the copolymerisation of these acids with other unsaturated aliphatic monomers for example vinyl monomers, such as vinyl hydrocarbon monomers, vinyl ethers, acrylamide or acrylonitrile. Particularly noteworthy are the homopolymers of acrylic acid and its copolymers, particularly copolymers of acrylic acid and itaconic acid, especially those described and claimed in UK 1484454. Good results have also been obtained using a copolymer of vinyl methyl ether and maleic acid. Examples of homo- or co-polymers of an unsaturated sulphonic acid include those prepared by the homopolymerisation and copolymerisation of ethylene sulphonic acid.

It is also possible to use a hydrolysable precursor of such polymers, for example a poly(carboxylic acid anhydride); furthermore, polyacrylic acids may be prepared by hydrolysis of corresponding polyacrylonitriles. The hydrolysable precursor of a poly(carboxylic acid) may be a homopolymer of an unsaturated carboxylic acid anhydride or a copolymer with an above-mentioned other carboxylic acid or anhydride thereof, or a copolymer of an unsaturated carboxylic acid anhydride with an unsaturated aliphatic monomer, for example vinyl monomers, such as vinyl hydrocarbon monomers, linear or cyclic vinyl ethers, acrylamide or acrylonitrile, for example pyran copolymer. Good results may be obtained by using homopolymers of maleic anhydride or vinyl orthophthalic anhydride, or copolymers thereof, especially block copolymers thereof, with ethylene, propylene, butenes, styrene and vinyl methyl ether. Mixtures of such components may be used. Preferably, the acid cement-forming component is in aqueous solution.

The acid-base reaction cement is also suitably formed from a base cement-forming component which comprises a basic or amphoteric oxide or hydroxide, or a salt of a weak or volatile acid. There are many basic or amphoteric oxides or hydroxides which can form cements with at least one of the acid-cement forming components defined above; examples include $Li_2O$ (other Group IA oxides or hydroxides tend to give materials which are too soluble in aqueous media, although they can be incorporated in minor amounts to facilitate release in other cements), Group IIA oxides, preferably calcined, such as MgO, "Ti(OH)$_4$", "Zr(OH)$_4$", $V_2O_5$, $Fe_2O_3$, $Cu_2O$, CuO, ZnO, preferably calcined, $Al_2O_3$ x $H_2O$ and SnO. Salts of weak or volatile acids include carbonates, monocarboxylates, such as acetates, and halides, such as the halides of Mg, Ca, Ba, Th, Ti, Zr, Al and Sn. They also include the extensive class of monomeric and polymeric (alumino)silicates, (alumino)phosphates and (alumino)borates which include the acid-reactive natural and synthetic minerals and ion-leachable glasses. By "(alumino)-silicate" is meant herein a silicate or an aluminosilicate; by "(alumino)phosphate" is meant herein a phosphate or an aluminophosphate; by "(alumino)borate" is meant herein a borate or an aluminoborate. Examples of ion-leachable glasses include those glasses wherein the principal acidic oxide is silica (although the glass may also contain minor amounts of other anhydrides such as phosphorus pentoxide and boric oxide), and wherein the principal basic oxide in the glass is alumina which, although it has amphoteric properties, can be considered for the purposes of the present invention solely as a basic oxide. Particularly preferred glasses fall within the composition range of 10 to 65% w/w silica and 15 to 50% w/w alumina. The glass desirably contains at least one other basic oxide, preferably calcium oxide, which may be present in the glass composition in an amount from 0 to 50% w/w. The calcium oxide may be partly or wholly replaced by sodium oxide or other basic oxide or a mixture of basic oxides. The presence of sodium oxide can be desirable in increasing the solubility of the resulting cement. Preferred glasses for use in the present invention containing alumina, silica and calcium oxide are the gehlenite and anorthite glasses, and in general glasses falling within the composition range 10 to 65% w/w silica, 15 to 50% w/w alumina and 0 to 50% w/w calcium oxide.

Other glasses suitable for use in the present invention may contain fluoride, suitably up to 15% by weight, preferably less than 10% by weight. A class of fluoraluminosilicate glasses particularly suited to this invention are those wherein the ratio by weight of silica to alumina is from 1.5 to 2.0 and the ratio by weight of fluorine to alumina is from 0.6 to 2.5, or wherein the ratio by weight of silica to alumina is from 0.5 to 1.5 and the ratio by weight of fluorine to alumina is from 0.25 to 2.0.

Mixtures of such components may be used.

It is noted that, apart from cement-forming components of unequivocal acidity or basicity, certain components may react as acid cement-forming components under a given set of reaction conditions while reacting as base cement-forming components under a different set of reaction conditions.

Specific classes of substance having a medicinal effect which may be utilised in a sustained release device of the invention include hypnotics, sedatives, tranquilisers, antipyretics, antiinflammatory agents, antihistamines, antitussives, anticonvulsants, muscle relaxants, topical or dermatological agents, antispasmodics, anti-ulcer agents, preparations containing various substances for the treatment of infection by pathogens including anti-fungal agents, antiparasitic agents such as anthelmintics; for example avermectin and morantel tartrate, and other antimicrobials such as antibiotics; for example oxytetracycline, preparations containing hormones, for example androgenic, estrogenic and progestational hormones, notably steroids, such as progesterone, trenbolone, oestradiol and polypeptide hormones, sympathamimetic agents, hypoglycaemic agents, nutritional agents, preparations containing enzymes of various types of activity, for example chymotrypsin, preparations containing analgesics, for example aspirin and agents with many other types of action including nematocides and other agents of veterinary application. Mixtures of active substances may be incorporated into the sustained release device of the invention which may suitably comprise a dosage form, especially one which is an integral body of cement.

In order to ensure retention of oral dosage sustained release devices of this invention in the reticulo-rumen of ruminant stock, it is desirable to incorporate therein a weighting agent to raise the density of the device, typically to about 3, although a somewhat lower density may be appropriate. The weighting agent may be dispersed throughout the device (e.g. as iron filings) or consolidated (e.g. as an essentially coaxially positioned steel rod).

The configuration of the sustained release devices of the invention is selected having regard both to the desired release characteristics and ease of administration. The devices are most often used in the form of a shaped body such as a hollow or blank cylinder, a sphere, a tablet or a slab and the nature of the shape and its dimensions may be selected appropriately. A primary target is to achieve a sustained release over appropriate time period, conveniently of a major proportion, for example 80 or 90%, of the active substance. Unusual release profiles may, however, be obtained by utilising devices which comprise open cavities, for example hollow cylinders or slabs with one or more holes or hollows in them. It is found that the release profiles of such devices can go through a maximum with time. Such geometric control of release profiles provides very useful additional means of obtaining and controlling improved release profiles. It is preferred, however, that the device is a cylindrical dosage form which may be mono- or bis- spherically capped. For administration orally to sheep this may be up to 20mm in diameter and 50mm long; for administration orally to cattle this may be up to 35mm in diameter and 160mm long. For parenteral administration either to sheep or cattle cylinders 3mm in diameter and 5mm long are very suitable. Up to 4 in sheep and 10 in cattle may be incorporated in one implant.

The cement can be formulated to have either a stable or an erodible matrix and this is one of the factors that govern the kinetics of release of the active species. The species can be a constituent of the cement matrix itself or added as a third component.

Because the cements are formed at room temperature it is perfectly possible to incorporate an organic species into the cement. This is an advantage over glass matrices, where obviously organic species cannot be incorporated into at the time of preparation.

An erodible matrix is suitable for the release of insoluble organic species when they are an essential constituent of the cement. Soluble species can be released either from a stable or an erodible matrix. In the former situation the release will most probably be governed by diffusion.

Drugs can be incorporated easily into these matrices, by adding them during the preparation of the cement before it was gelled.

The invention will now be described by way of example. The drawings are further explained in Example 40 later.

| | | | Cement | | |
|---|---|---|---|---|---|
| Drug | GI | DS | ZPC | MOS | CHC |
| Progesterone | 1 | 2 | 3 | 4 | 5 |
| Indomethacin | - | 6 | 7 | 8 | 9 |
| Chloroquine | - | - | - | - | - |
| Nalidixic acid | 10 | 11 | 12 | 13 | 14 |
| Theophylline | 15 | 16 | 17 | 18 | 19 |
| Isoniazid | - | 20 | 21 | 22 | 23 |
| L-Dopa | - | 24 | - | 25 | 26 |
| Morphine | 27 | 28 | 29 | 30 | 31 |
| Ibuprofen | 32 | 33 | 34 | 35 | 36 |
| Ranitidine | - | - | - | 37 | - |
| Aspirin | 38 | 39 | - | - | - |
| Amprolium | - | - | - | 40 | - |
| p-Nitrophenol | 41 | 42 | 43 | 44 | 45 |

GI = glass ionomer (aluminosilicate glass base + poly(acrylic acid))

DS = dental silicate (aluminosilicate glass base + phosphoric acid)

ZPC = zinc polycarboxylate (zinc oxide base + poly(acrylic acid))

MOS = nominally magnesium oxysulphate (magnesium oxide base + magnesium sulphate as acid) - in aqueous media this cement tends to lose magnesium sulphate to become magnesium hydroxide.

CHC = calcium hydroxide chelate (calcium hydroxide base + salicylic ester as acid)

Examples 1-45 are all according to the invention. The combinations without a number are not according to the invention, and the reasons are varied.

Ranitidine delayed the setting of the aluminosilicate cements and accelerated that of the calcium hydroxide cements. The introduction of indomethacin induced hydrolytic instability of the glass-ionomer.

Chloroquine prevented the dental silicate and glass-ionomer cements from setting and introduced some hydrolytic instability into the zinc polycarboxylate cement. No suitable cement matrix was found for this drug.

The two alkaline cements (the calcium hydroxide chelate cement and the magnesium oxysulphate cement) degraded Aspirin, Chloroquine and L-Dopa to a considerable extent. The glass-ionomer and zinc polycarboxylate cements caused slight degradation of Ranitidine and L-Dopa. The zinc polycarboxylate cement also degraded Chloroquine to some extent.

There was rapid release of amprolium from the magnesium oxychloride cement and the drug was completely eluted in less than 2000 minutes. Release was much less rapid from zinc polycarboxylate and zinc phosphate cements and appeard to follow classical diffusion kinetics, i.e. the initial rate of release was proportional to $t^{-1/2}$, and the initial cummulative release profile was proportional to $t^{1/2}$. Neither profile is ideal for the sustained release of a drug.

Further information on release profiles for a selected example is as follows:

Example 40

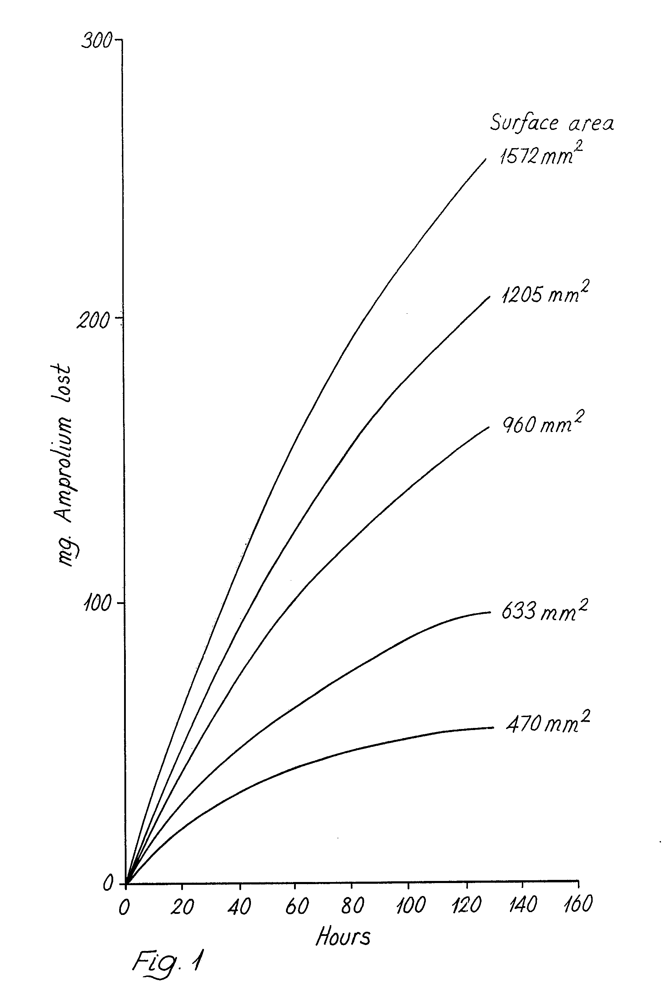

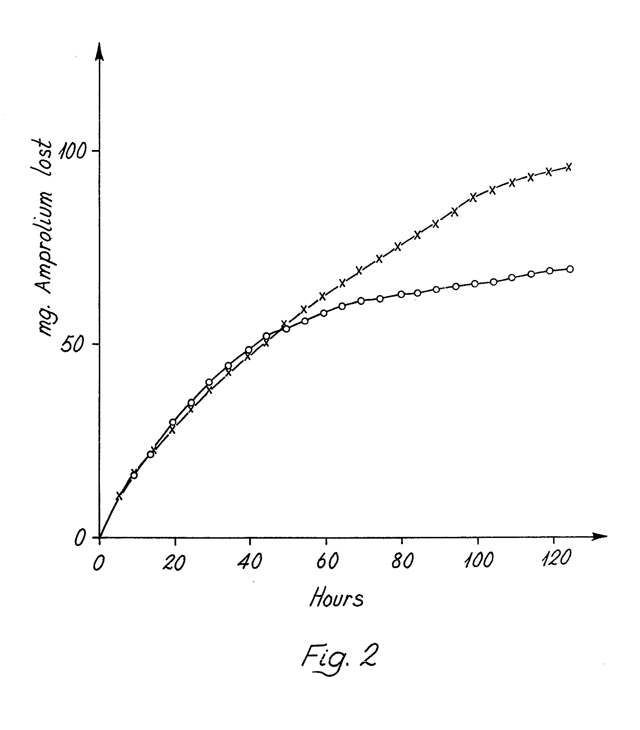

The release profile from magnesium oxysulphate cement proved to be superior to that from other cements especially for amprolium. Amprolium was monitored by its absorption of 267nm radiation. The release rate had both diffusion ($t^{-1/2}$) and zero order terms, the latter becoming, obviously, more dominant with time until the cement was depleted of the drug. The rate of release was proportional to the surface area of the device and not its volume. This is further shown in Figure 1, wherein cumulative loss curves for magnesium oxysulphate cylindrical boluses containing 5 weight % amprolium are plotted for five boluses, all 13mm-diameter cylinders, but of lengths 4,9,17,23 and 32mm. The abcissa is hours in a dissotester, viz. a vessel wherein the bolus was leached by demineralised water at 37C passed at 1ml/minute. The ordinate is cumulative mg of amprolium lost from the bolus. The cumulative release of the drug from two devices of the same surface area but different volumes was studied. Initially, the rate of release was identical; however, differences later were observed as the cement became depleted of the drug. This is further shown in Figure 2, wherein cumulative loss curves for two magnesium oxysulphate cylinders containing 5 weight % amprolium are plotted. The curve x-x is of a 13mm diameter 9mm deep cylinder (volume 1195mm$^3$) while o-o is of a $5\frac{1}{2}$ mm diameter 34mm deep cylinder (volume 808mm$^3$), both having the identical surface area. The abcissa is as explained for Figure 1; after 120 hours, the x-x cylinder had lost 95% of its amprolium and the o-o cylinder had lost 100%. The ordinate is as in Figure 1.

**Claims**

1. A sustained release device, comprising a shaped cementitious mass or a tablet formed of crushed cementitious material, the cement being an acid-base reaction cement and comprising a pharmaceutically active organic substance.

2. A sustained release device according to Claim 1, wherein the cement is formed from an acid cement-forming component which comprises a mineral acid, an acid salt, a polyfunctional organic phosphoric acid, a polyfunctional phenol, a homo- or co-polymer of an unsaturated carboxylic or sulphonic acid, or a hydrolysable precursor thereof.

3. A sustained release device according to Claim 1 or Claim 2, wherein the base cement-forming component comprises a basic or amphoteric oxide or hydroxide or salt of a weak or volatile acid.

4. A sustained release device according to Claim 3, wherein the oxide is an ion-leachable glass.

5. A sustained release device according to Claim 3, wherein the cement is a magnesium oxysulphate cement.

6. A sustained release device according to any preceding claim, wherein the organic substance is amprolium.

7. A sustained release device according to any preceding claim, having incorporated therein a weighting agent to increase the density of the device.

8. A sustained release device according to Claim 1 substantially as hereinbefore described with reference to any one of Examples 1 to 45.

Fig. 1

Fig. 2